# EUROPEAN PATENT SPECIFICATION

(11) **EP 1 395 603 B1**
(45) Date of publication and mention of the grant of the patent: **10.03.2010**
(21) Application number: 02735607.0
(22) Date of filing: 11.06.2002
(51) Int. Cl.: C07J 5/00, C07J 3/00, C07J 71/00

(54) **PREPARATION OF FLUMETHASONE AND ITS 17-CARBOXYL ANDROSTEN ANALOGUE**
HERSTELLUNG VON FLUMETHASONE UND DESSEN 17-CARBOXYL ANDROSTEN ANALOG
PREPARATION DU FLUMETHASONE ET DE SON ANALOGUE 17-CARBOXYLE ANDROSTENE

(30) Priority: 12.06.2001 PT 10262801
(43) Date of publication of application: 10.03.2004
(73) Proprietor: Hovione Limited ., Whanchai, Hong Kong (CN)
(72) Inventor: VILLAX, Ivan, P-1200-671 Lisboa (PT); MENDES, Zita, P-1000-260 Lisboa (PT)
(74) Representative: Wain, Christopher Paul
(86) International application number: PCT/GB2002/002644
(87) International publication number: WO 2002/100878

(56) References cited:
- EP-A- 0 610 138
- EP-A- 1 207 166
- US-A- 4 188 322
- US-A- 4 255 331
- DATABASE FILE REGISTRY [Online] Chemical abstracts (STN); XP002207533

## Description

This invention relates to a process for preparing flumethasone, flumethasone 21-acetate and its 17-carboxyl androsten analogue, and to certain starting materials for the process.

Flumethasone, 6α,9α-difluoro-16α-methylprednisolone was described for the first time in 1962. Although this corticosteroid has an enhanced anti-inflammatory activity, it has not been widely used clinically. At the present time, its economical preparation on an industrial scale is ever more important because it is also an excellent starting material for the production of new difluoro-17-carboxyl androstenes, which are becoming increasingly important from a clinical point of view.

Flumethasone and its production are the subject of a number of patents including US Patent 3,499,016 (1962) and British Patent 902,292 (1970). The new synthetic techniques developed since 1970 naturally permit a more efficient production of flumethasone with considerably increased yields compared to those obtained with the initial patents.

We have now devised a new process for the preparation of flumethasone as well as to the preparation of 6α,9α-difluoro-11β,17α-dihydroxy-16a-methyl-17β-carboxy-androsta-1,4-diene-3-one which is also called "hydroxyacid", an excellent starting material for the production of fluticasone and other new anti-inflammatory compounds of the androsta-1,4-diene series. The "hydroxyacid" was first described and claimed in US Patent 3,636,010 (priority 1968).

European Patent 0 610 138 B1 (1994) describes a new synthetic route for the preparation of the so called "hydroxyacid". However, the present invention represents considerable unexpected advantages in relation to this prior process patent, namely:
- the reaction sequence is reduced by one reaction step and by the elimination of the desolvatation step of 6α,9α-difluoro-11β,17α-dihydroxy-16α-methyl-17β-methoxycarbonyl androsta-1,4-diene-3-one, an additional production step,
- the present process avoids the use of the highly toxic reagent dimethyl sulphate,
- permits the simultaneous deacetylation and degradative oxidation of the pregnane side chain forming directly the equivalent androstan derivative,
- increased yield of the hydroxyacid with excellent purity.

Whilst all the reaction steps of the present invention are realised in the pregnane series, except the last one, thus permitting an efficient preparation of flumethasone, the reaction sequence of EP 0 610 138 B1 transforms the common starting material of both processes as from the first step into the androstane series.

According to the present invention, there is provided a process for preparing flumethasone (6α,9α-difluoro-11β,17α,21-trihydroxy-16α-methyl-pregna-1,4-diene-3,20-dione), flumethasone 21-acetate or its 17-carboxyl androsten analogue of the formula: 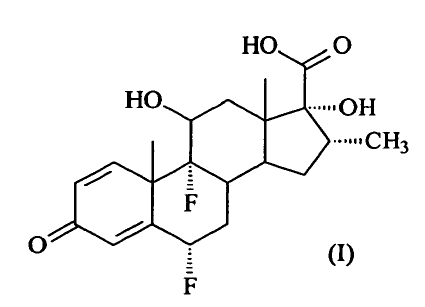 which process comprises
(a) reacting a compound of formula (II) 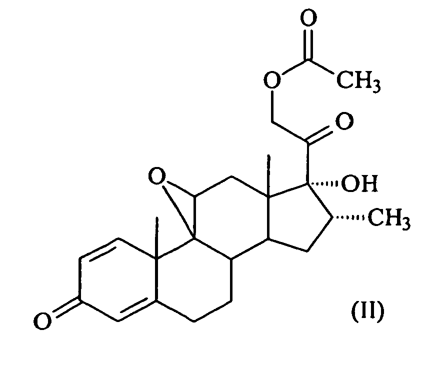 with benzoyl chloride to form a 3-enolic ester of the formula (IIIa): 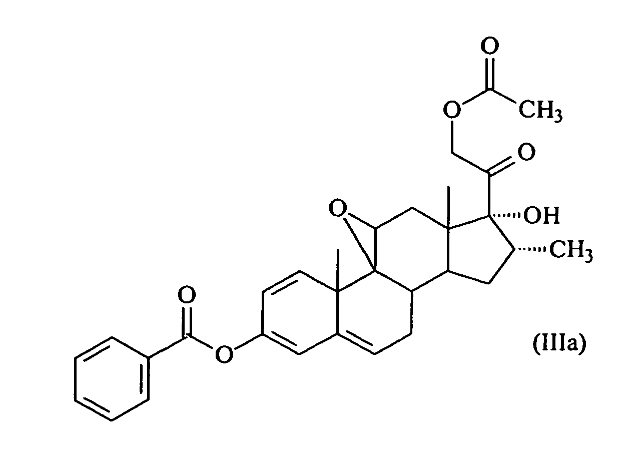
b) reacting the enol benzoate (IIIa) with an electrophilic fluorination agent to introduce fluorine in the C6-position to form a new compound of formula (IIIb): 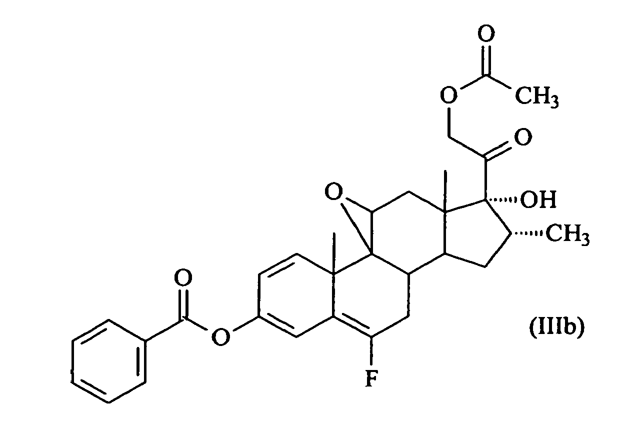
(c) deprotecting the compound (IIIb) at C3 to form a compound of formula (IV): 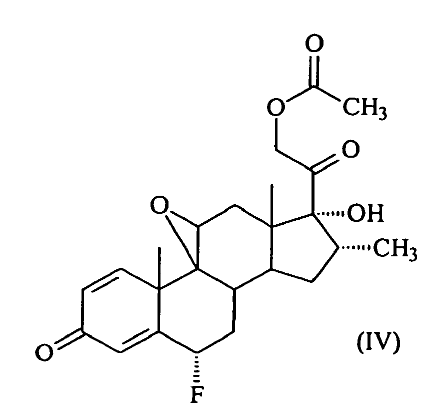
(d) fluorinating the 9,11-epoxy group of compound IV by reacting it with hydrofluoric acid to yield flumethasone 21-acetate; and
(e) optionally hydrolysing the flumethasone 21-acetate, in the presence or absence of an oxidising agent, to yield compound (I) or flumethasone, respectively.

The invention also provides compounds of the formula (V): 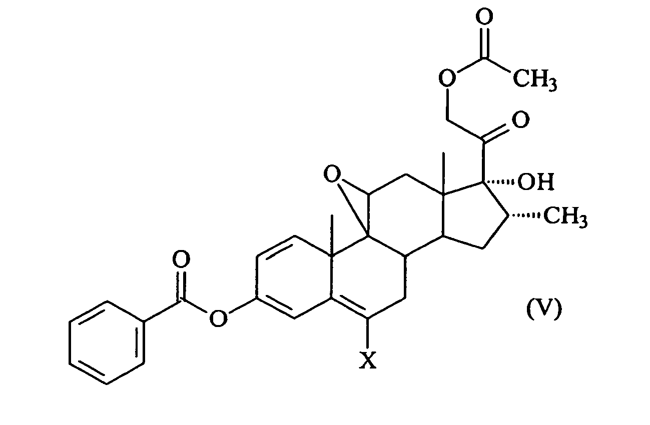 wherein X is hydrogen or fluorine.

In step (d) of the process, the flumethasone 21-acetate formed has the formula: 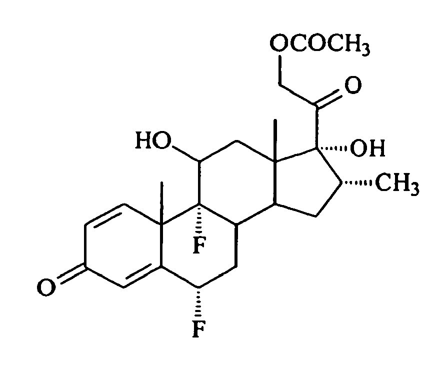 ie 6α,9α-difluoro-11β,17α,21-trihydroxy-16α-methyl-pregna-1,4-diene-3,20-dione, 21-acetate. If this compound is hydrolysed, preferably with methanolic potassium hydroxide, the flumethasone free alcohol is formed.

Alternatively, if the flumethasone acetate is hydrolysed, preferably with methanolic potassium hydroxide, and then oxidised, preferably with hydrogen peroxide solution, the so called "hydroxyacid" is obtained, of formula:

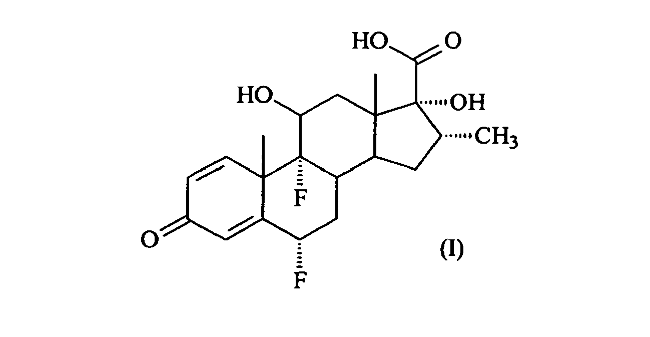

The present invention permits the direct transformation of flumethasone acetate into compound I.

The two new compounds IIIa and IIIb are depicted as a general formula (V) in claim 8.

The compound I can also be obtained as described in US Patent 3,636,010 by oxidizing flumethasone free alcohol.

The starting material of the present process is commercially available and widely used in the preparation of corticosteroids such as dexamethasone and icomethasone.

In order to introduce fluorine in the C6 position by electrophilic fluorination, it is necessary to activate first the C6 position. For that purpose, the 3-keto group is enolised by a carboxylic acid chloride, forming an enolic ester residue of the formula -COR in which R is a phenyl group. The compound for the enolisation is benzoyl chloride, yielding the compound of formula IIIa in the presence of a tertiary amine, such as pyridine. The preferred solvent is N,N'-dimethylacetamide and the reaction is preferably carried out at a temperature of 80 to 85°C, yielding the ? 3,5 enol benzoate. Thereafter, the compound IIIa is reacted with an electrophilic fluorination agent to yield the corresponding C6-fluoro derivative. The preferred fluorination agent is the 1-(chloromethyl)-4-fluoro-1,4-diazoniabicyclo[2.2.2]octane bis(tetrafluoroborate), Selectfluor^{®}. So as to realise the fluorination at C6, the preferred solvent is acetonitrile in presence of water at a preferred temperature of -5°C ± 2°C. After the C6 fluorination, the 3-enolic ester can be easily transformed into the system of 3-keto-1,4-diene yielding the compound IV. The elimination of the enolic ester is preferably effected by an aqueous solution of sodium metabisulfite and ammonia.

In the next step, a 9,11-epoxy group of compound IV is reacted with a concentrated aqueous solution of hydrofluoric acid or with a solution of hydrogen fluoride in N,N'-dimethylformamide by *per se* known processes at a temperature below 25°C. When compound IV is practically completely reacted, the reaction mixture is poured into a mixture of ice and ammonia sufficient to neutralise the hydrofluoric acid and precipitate simultaneously the flumethasone 21-acetate with a high yield and purity. The product obtained can be recrystallized for instance from methanol. The 21-acetate obtained can be subsequently hydrolyzed by any of the known processes yielding flumethasone free alcohol. One of the preferred processes is effected in degassed methanolic potassium hydroxide at a temperature between -15°C and -5°C. The end of the reaction is ascertained by HPLC after one hour and it is considered complete when the amount of starting compound remaining is less than 1 %.

In order to carry out the degradative oxidation, according to prior art, flumethasone is suspended in tetrahydrofuran and a solution of the oxidising agent is added dropwise. The substrate first starts to dissolve followed by precipitation. The oxidation is performed preferably at 20°C employing, for example, periodic acid. After one hour of stirring, completion of the reaction is determined by HPLC. Once the amount of non-reacted flumethasone is less than 0.3%, the reaction is considered complete. Subsequently, the reaction mixture containing compound I is precipitated by adding the reaction mixture to an aqueous solution of sodium metabisulfite and ice.

According to the present invention, flumethasone 21-acetate can be simultaneously deacetylated and oxidised by methanolic potassium hydroxide and aqueous hydrogen peroxide yielding, after completion of the reaction, the desired hydroxyacid, compound I, by acidifying the reaction mixture with diluted hydrochloric acid to pH 2. This reaction is performed at 10°C ± 2°C with agitation until the reaction is complete.

The cumulative stoichiometric yield of the process as described in EP 0 610 138 B1 so as to obtain unrecrystallized compound I is 48.9% from 9,11β-epoxy-17α,21-dihydroxy-pregna-1,4-diene-3,20-dione. According to the present process, however, the cumulative stoichiometric yield obtained is 62.4%, as per Examples 1b, 2 and 4, as from the 21-acetate of the above starting material. So as to obtain a valid comparison of yields, the starting material of EP 0 610 138 B1 has been first acetylated with a yield of 110% w/w and the cumulative stoichiometric yield was calculated on basis of this value and of Examples 1b, 2 and 4, resulting in 61.7%.

The following Examples serve to illustrate the present invention without limiting its scope.

### EXAMPLE 1

### 9,11β-epoxy-6α-fluoro-17α,21-dihydroxy-16α-methyl-pregna-1,4-diene-3,20-dione, 21-acetate (Formula IV)

a) 50 g of 9,11β-epoxy-17α,21-dihydroxy-16α-methyl-pregna-1,4-diene-3,20-dione, 21-acetate was placed under an inert atmosphere and dissolved in 25 ml of N,N'-dimethylacetamide. 65 ml of pyridine was added and the reaction mixture heated with stirring to a temperature between 80°C and 85°C. 33 ml of benzoyl chloride was added and the reaction mixture protected from light. The reaction was left to stir for two to three hours at this temperature. On completion of the reaction after this time, it was cooled at 40°C and 75 ml of methanol added with continued stirring at 40°C for a further 30 minutes followed by cooling the reaction mixture to 20-25°C. Subsequently, the reaction mixture was added to 1000 ml of water containing 57.5 ml of hydrochloric acid and 100 ml of dichloromethane. The phases were separated and the aqueous phase further extracted with an additional 100 ml of dichloromethane. The organic phase was washed with water and with an aqueous solution of sodium hydroxide. The dichloromethane solution thus obtained was evaporated to dryness under vacuum yielding an oil, 3-benzoyloxy-9, 11β-epoxy-17α,21-dihydroxy-16α-methyl-pregna-1,3,5-triene-20-one 21-acetate (Formula IIIa). 3-Benzoyloxy-9,11β-epoxy-17α,21-dihydroxy-16α-methyl-pregna-1,3,5-triene-20-one 21-acetate was taken up by 150 ml of acetonitrile cooled to between -5°C and 0°C. The solution of the enolbenzoate was added to a suspension of 44.5 g of 1-(chloromethyl)-4-fluoro-1,4diazoniabicyclo[2.2.2]octanebis(tetrafluoroborate), Selectfluor®, in 175 ml of acetonitrile containing 5 ml of water.
   Once the fluorination reaction at C6 was complete (Formula IIIb), the reaction mixture was poured into a solution of 100 ml water, 1.2 g of sodium metabisulfate, 5 ml of ammonia (25%) and 200 ml of dichloromethane. The pH of the solution was adjusted to between 7-8 and stirred for 30 minutes after which the phases were separated and the organic phase washed with ammonia solution (12.5%). The organic phase was evaporated to dryness under vacuum and methanol added. The desired compound crystallized which was then filtered and dried at 40 to 45°C, yielding 40 g of the title product with a purity of 90% in area as determined by HPLC.
b) Example 1a was repeated, however, the 3-enol benzoate formed was not extracted and isolated. To the reaction mixture obtained in Example 1a, 44.5 g of Selectfluor^{®} crystals were slowly and directly added in four portions. When the content of the starting material was determined to be less than 1%, the reaction mixture was poured into 100 ml of water containing 1.2 g of sodium metabisulfite. The pH was adjusted to between 7 and 7.5, the solution stirred for 30 minutes, and the precipitate filtered and washed. The product thus obtained was washed by suspending it in 150 ml of methanol with stirring. After stirring for 30 minutes, it was filtered and dried at a temperature between 40-45°C, yielding 46 g of 9,11β-epoxy-6α-fluoro-17α,21-dihydroxy-1,4-diene-3,20-dione, 21-acetate with a purity of 91.6%.

### EXAMPLE 2

### 6α,9α-difluoro-11β,17α,21-trihydroxy-16α-methyl-pregna-1,4-diene-3,20-dione, 21 acetate (Flumethasone 21-acetate).

36 g of the compound obtained in Example 1 was dissolved in 360 ml of a complex of hydrogen fluoride and N,N'-dimethylformamide (∼64% w/w) under an inert atmosphere and at a temperature of 20°C ± 3°C. After stirring for three hours at this temperature it was poured, with agitation, into a mixture of 3000 ml of water, 1000 ml of ice and 800 ml of ammonia (25%) where the temperature was maintained below 25°C during the whole precipitation process. The pH was adjusted to between 4.5 to 5 with ammonia solution and the agitation continued for one hour. After this time, the precipitate was filtered and washed with water until neutral pH was achieved. The compound was dried, after which it was dissolved in a mixture of 333 ml of dichloromethane and 148 ml of methanol. The solution was concentrated to a volume of 89 ml and the desired compound crystallised. After filtration, it was dried at a temperature between 40 to 45°C yielding 29.2 g of 6α,9α-difluoro-11β,17α,21-trihydroxy-16α-methyl-pregna-1,4-diene-3,20-dione, 21-acetate with a purity of 95% in area, as determined by HPLC.

### EXAMPLE 3

### 6α,9α-difluoro-11β,17α,21-trihydroxy-16α-methyl-pregna-1,4-diene-3,20-dione (Flumethasone)

1.4 g of potassium hydroxide was dissolved in 140 ml of degassed methanol under an inert atmosphere and the solution cooled to between 0°C and -5°C. Subsequently, the solution was added with stirring to a suspension of 28 g of the compound obtained in the previous example to 700 ml of degassed methanol. The reaction was stirred for one to two hours at a temperature of -10°C ± 2°C. Once the reaction was complete, as determined by HPLC, acetic acid was added until pH 7 was achieved. The volume of the reaction mixture was reduced under vacuum to ∼224 ml. It was then cooled to 10°C and 140 ml of cold water added. After stirring for one hour at a temperature of between 5°C to 10°C, the compound was filtered, washed with water and dried at 45°C, to yield 22.4 g of the title compound with a purity of 96% in area as ascertained by HPLC.

### EXAMPLE 4

### 6α,9α-difluoro-11β,17α-dihydroxy-16α-methyl-17β-carboxy-androsta-1,4-diene-3-one (Formula I, "hydroxyacid")

2 g of potassium hydroxide was dissolved in a mixture of 100 ml of methanol and 100 ml of water under an inert atmosphere. 10 ml of an aqueous hydrogen peroxide solution (130 vol.) was subsequently added and the reaction mixture cooled to 10°C ± 2°C, upon which 5 g of flumethasone 21-acetate was added. The reaction was stirred overnight at this temperature and on completion of the reaction, the pH was adjusted to 2 with hydrochloric acid. The precipitate obtained was filtered, washed with water until neutral pH was obtained and dried at 45°C. The yield of the title compound was 80% w/w, corresponding to a stoichiometric yield of 91.3%.

### EXAMPLE 5

### 6α,9α-difluoro-11β,17α-dihydroxy-16α-methyl-17β-carboxy-androsta-1,4-diene-3-one.

22 g of flumethasone free alcohol, as obtained in Example 3, was suspended in 110 ml of tetrahydrofuran under an inert atmosphere and cooled to 20°C ± 2°C. 17.6 g of periodic acid in 70 ml of water was slowly added with stirring. After stirring at this temperature the end of the reaction was ascertained by HPLC. Generally the reaction was found to be complete after two hours. Subsequently, the reaction mixture was poured into a solution of 33 g of sodium metabisulfite in 770 ml of water and 330 ml of ice. On precipitation of the product, it was filtered, washed with water until neutral pH and dried at 40°C to 45°C to yield 21 g of the title compound with a purity of 96% in area, as ascertained by HPLC. After recrystallization of the thus obtained compound in ethanol, 6α,9α-difluoro-11β,17α-dihydroxy-16α-methyl-17β-carboxy-androsta-1,4-diene-3-one was obtained in high purity and had the following analytical values:
- optical rotation = +64.4° (c=1% DMF)
- KF - 0.09%
- Purity by HPLC = 99.2% in area
- Principal absorption peaks in infrared at 1698 cm⁻¹, 1660 cm⁻¹, 1614 cm⁻¹ and 1603cm⁻¹.

## Claims

1. A process for preparing flumethasone (6α,9α-difluoro-11β,17α,21-trihydroxy-16α-methyl-pregna-1,4-diene-3,20-dione), flumethasone 21-acetate or its 17-carboxyl androsten analogue of the formula: 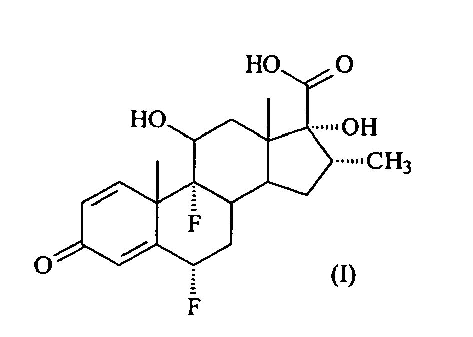 which process comprises
(a) reacting a compound of formula (II) 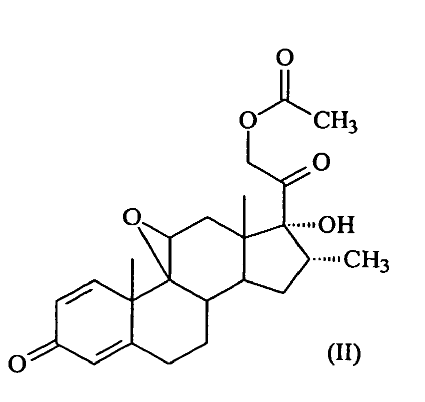 with benzoyl chloride to form a 3-enolic ester of the formula (IIIa): 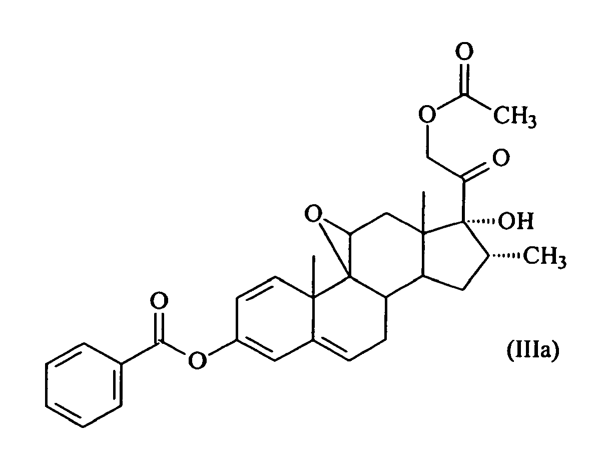
b) reacting the enol benzoate (IIIa) with an electrophilic fluorination agent to introduce fluorine in the C6-position to form a compound of formula (IIIb): 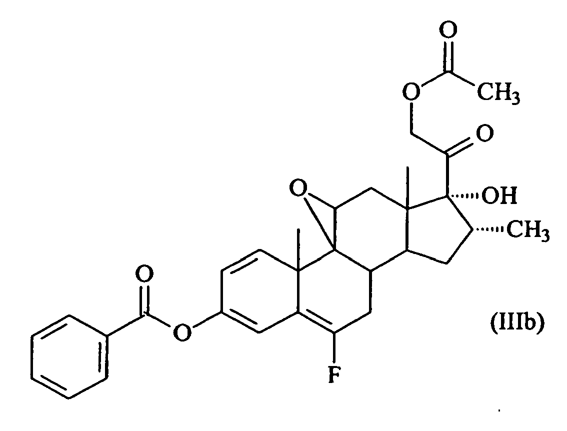
(c) deprotecting the compound (IIIb) at C3 to form a compound of formula (IV): 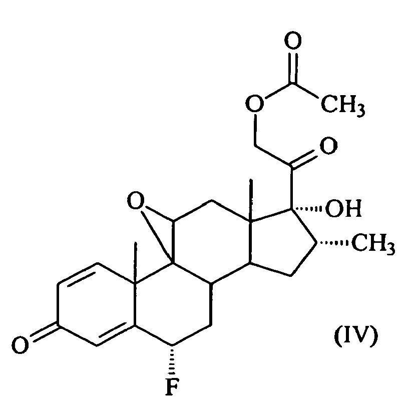
(d) fluorinating the 9,11-epoxy group of compound IV by reacting it with hydrofluoric acid to yield flumethasone 21-acetate; and optionally
(e) hydrolysing the flumethasone 21-acetate, in the presence or absence of an oxidising agent, to yield compound (I) or flumethasone, respectively.

2. A process according to claim 1 wherein, in step (e), the flumethasone 21-acetate is hydrolysed with methanolic potassium hydroxide.

3. A process according to claim 1 or 2, wherein in step (e) the oxidising agent is aqueous hydrogen peroxide solution.

4. A process according to claim 1, 2 or 3, wherein the electrophilic fluorination agent is 1-(chloromethyl)-4-fluoro-1,4-diazoniabicyclo[2.2.2]octane bis(tetrafluoroborate).

5. A process according to any of claims 1 to 4, wherein in step (c) the deprotection in position C3 is effected using an aqueous solution of metabisulfite and ammonia.

6. A process according to any preceding claim, wherein in step (a) the reaction medium is N,N'-dimethylacetamide and pyridine; and in step (b) the reaction medium is acetonitrile in the presence of water.

7. A process according to any of claims 1 to 6, wherein the reaction temperature in step (a) is from 80 to 85°C and in step (b) is -5°C ± 2°C.

8. A compound of the formula (V): 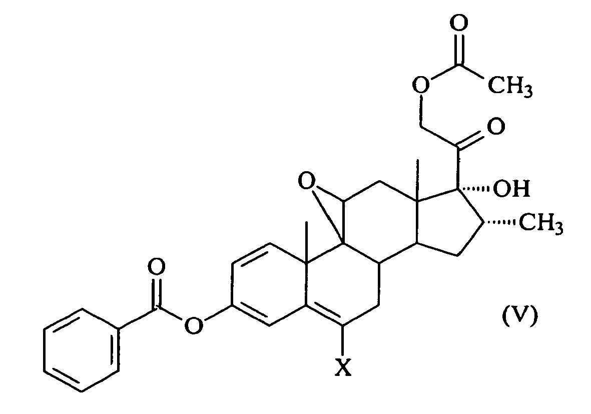 wherein X is hydrogen or fluorine.

## Patentansprüche

1. Verfahren zum Herstellen von Flumethason (6α,9α-Difluor-11β,17α,21-trihydroxy-16α-methyl-pregna-1,4-dien-3,20-dion), Flumethason-21-acetat oder seinem 17-Carboxyl-Androsten-Analogon der Formel: 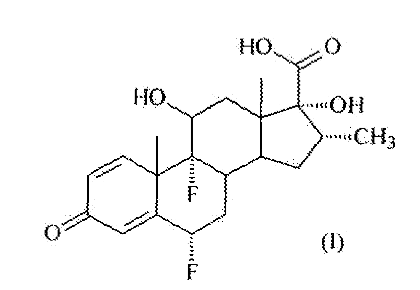 wobei das Verfahren umfasst
a) das Umsetzen einer Verbindung der Formel (II) 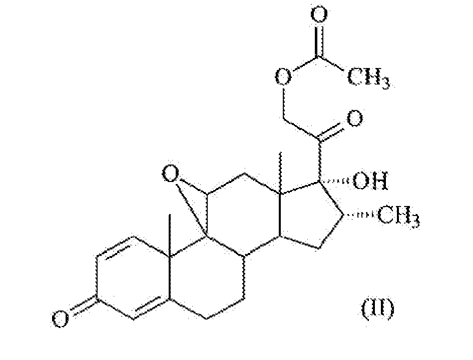 mit Benzoylchlorid, um einen 3-Enolester der Formel (IIIa) zu bilden: 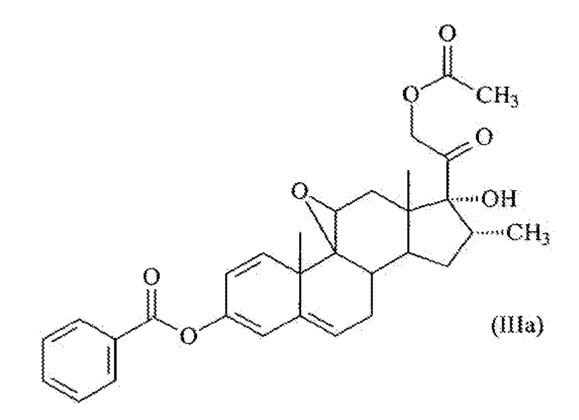
b) das Umsetzen des Enolbenzoats (IIIa) mit einem elektrophilen Fluorierungsmittel, um Fluor in die C6-Stellung einzuführen, um eine Verbindung der Formel (IIIb) zu bilden: 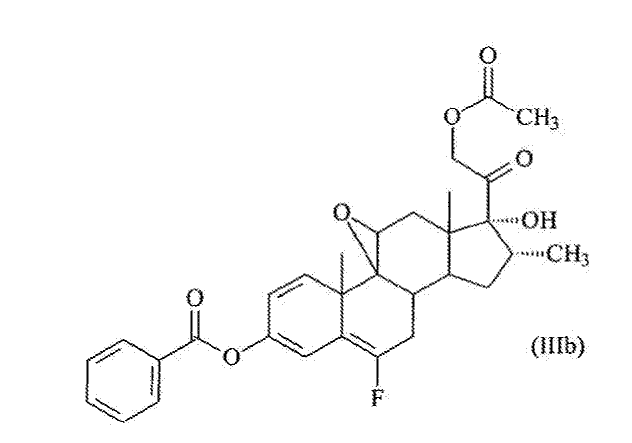
c) das Entschützen der Verbindung (IIIb) an C3, um eine Verbindung der Formel (IV) zu bilden: 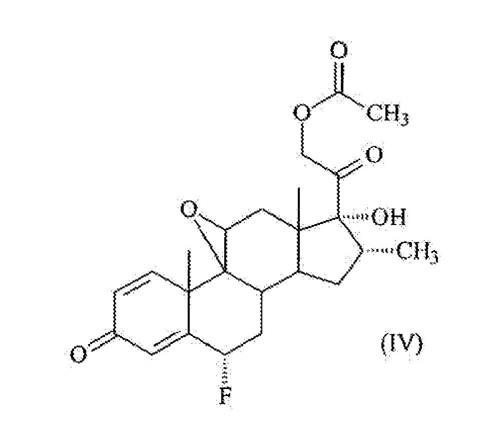
d) das Fluorieren der 9,11-Epoxygruppe der Verbindung IV durch Umsetzen dieser mit Fluorwasserstoffsäure, um Flumethason-21-acetat zu erhalten; und gegebenenfalls
e) das Hydrolysieren des Flumethason-21-acetats, in Gegenwart oder Abwesenheit eines Oxidationsmittels, um Verbindung (I) bzw. Flumethason zu erhalten.

2. Verfahren nach Anspruch 1, wobei in Schritt e) das Flumethason-21-acetat mit methanolischem Kaliumhydroxid hydrolysiert wird.

3. Verfahren nach Anspruch 1 oder 2, wobei in Schritt e) das Oxidationsmittel wässrige Wasserstoffperoxidlösung ist.

4. Verfahren nach Anspruch 1, 2 oder 3, wobei das elektrophile Fluorierungsmittel 1-(Chlormethyl)-4-fluor-1,4-diazoniabicyclo[2.2.2]octan-bis(tetrafluorborat) ist.

5. Verfahren nach einem der Ansprüche 1 bis 4, wobei in Schritt c) die Entschützung in Stellung C3 unter Verwendung einer wässrigen Lösung von Metabisulfit und Ammoniak bewirkt wird.

6. Verfahren nach einem der vorangehenden Ansprüche, wobei in Schritt a) das Reaktionsmedium N,N'-Dimethylacetamid und Pyridin ist; und in Schritt b) das Reaktionsmedium Acetonitril in Gegenwart von Wasser ist.

7. Verfahren nach einem der Ansprüche 1 bis 6, wobei die Reaktionstemperatur in Schritt a) 80 bis 85 °C beträgt und in Schritt b) -5 °C ± 2 °C beträgt.

8. Verbindung der Formel (V): 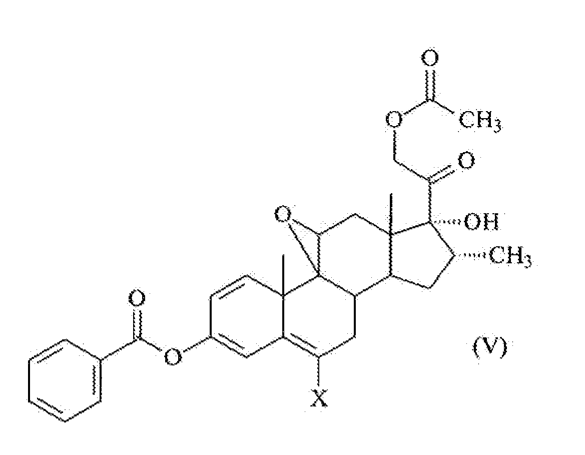 worin X Wasserstoff oder Fluor ist.

## Revendications

1. Procédé pour la préparation de fluméthasone (6α,9α-difluoro-11β,17α,21-trihydroxy-16α-méthyl-pregna-1,4-diène-3,20-dione), de fluméthasone 21 acétate ou de son analogue 17 carboxyle androstène de formule : 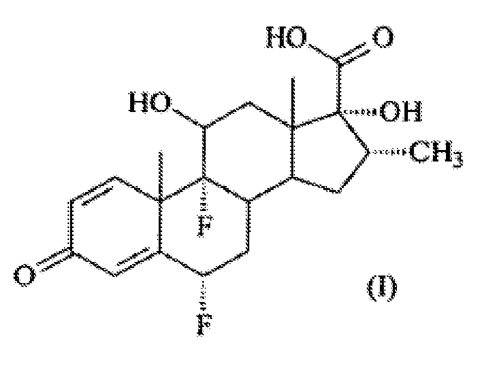 lequel procédé comprend les étapes consistant à
(a) faire réagir un composé de formule (II) 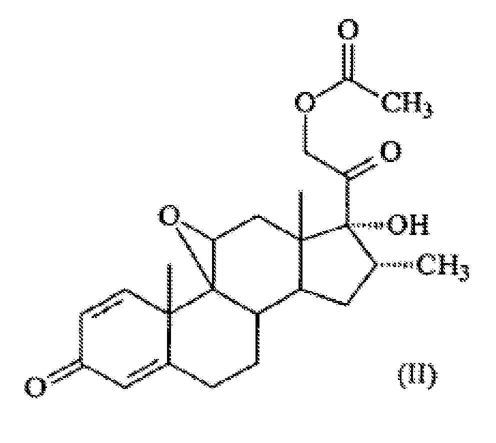 avec du chlorure de benzoyle afin de former un ester 3-énolique de formule (IIIa) : 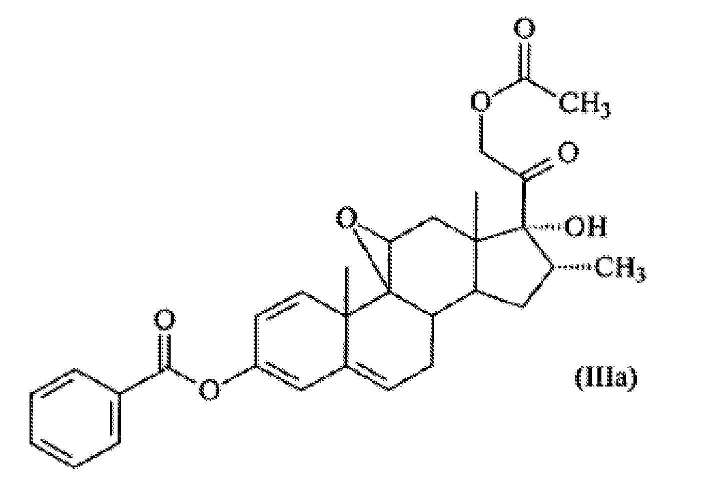
b) faire réagir le benzoate d'énol (IIIa) avec un agent de fluoration électrophile pour introduire du fluor en position C6 afin de former un composé de formule (IIIb) : 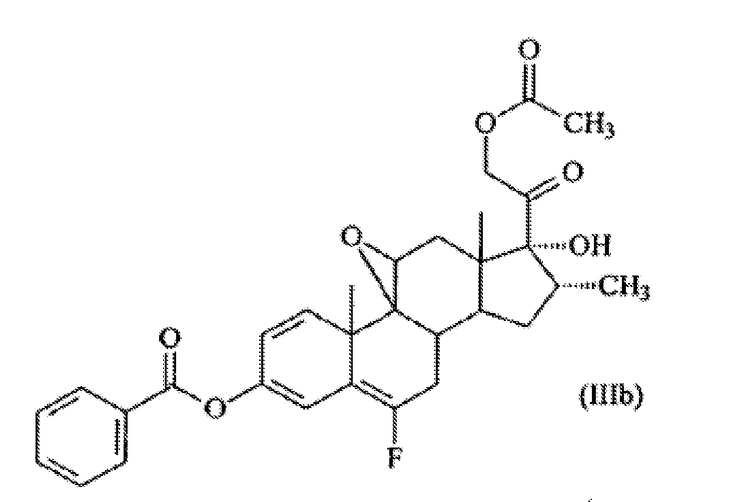
(c) déprotéger le composé (IIIb) en C3 afin de former un composé de formule (IV) : 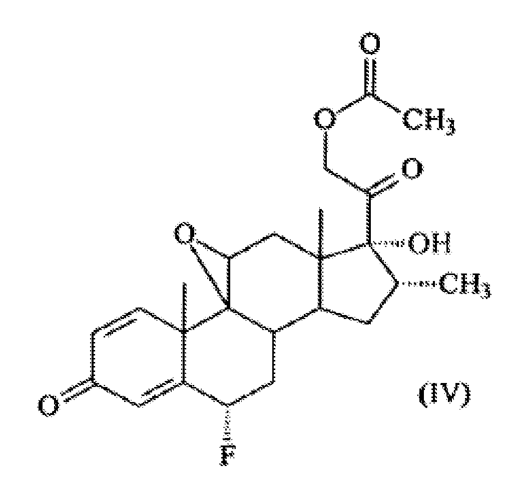
(d) fluorer le groupe 9,11-époxy du composé IV en le faisant réagir avec de l'acide fluorhydrique pour obtenir de la fluméthasone 21 acétate ; et éventuellement
(e) hydrolyser la fluméthasone 21 acétate, en présence ou en l'absence d'un agent oxydant, pour donner le composé (I) ou de la fluméthasone, respectivement.

2. Procédé selon la revendication 1, dans lequel, lors de l'étape (e), la fluméthasone 21 acétate est hydrolysée avec de l'hydroxyde de potassium méthanolique.

3. Procédé selon la revendication 1 ou 2, dans lequel lors de l'étape (e) l'agent oxydant est une solution de peroxyde d'hydrogène aqueux.

4. Procédé selon la revendication 1, 2 ou 3, dans lequel l'agent de fluoration électrophile est le 1-(chlorométhyl)-4-fluoro-1,4-diazoniabicyclo[2.2.2]octanebis (tétrafluoroborate).

5. Procédé selon l'une quelconque des revendications 1 à 4, dans lequel lors de l'étape (c) la déprotection en position C3 est réalisée en utilisant une solution aqueuse de métabisulfite et d'ammoniac.

6. Procédé selon l'une quelconque des revendications précédentes, dans lequel lors de l'étape (a), le milieu de réaction est le N,N'-diméthylacétamide et la pyridine ; et lors de l'étape (b) le milieu de réaction est l'acétonitrile en présence d'eau.

7. Procédé selon l'une quelconque des revendications 1 à 6, dans lequel la température de réaction lors de l'étape (a) va de 80 à 85 °C et lors de l'étape (b) de -5 °C à ± 2 °C.

8. Composé de formule (V) : 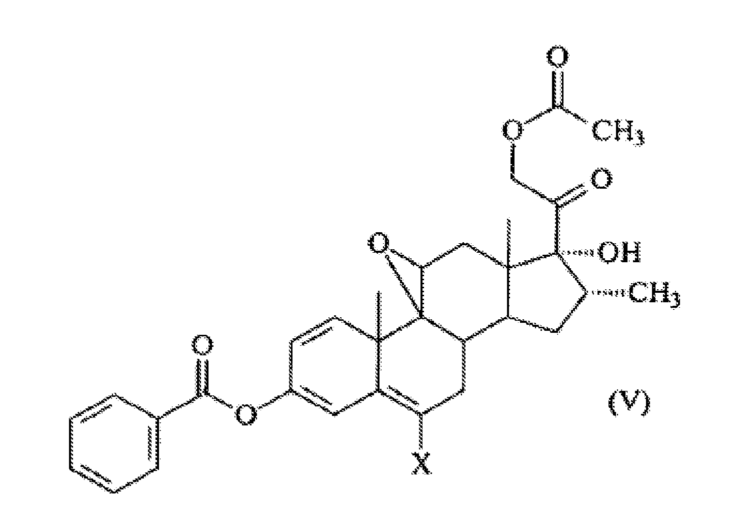 où X représente l'hydrogène ou le fluor.
